(19) Europäisches Patentamt
European Patent Office
Office européen des brevets

(11) Veröffentlichungsnummer: **0 240 829 B1**

# (12) EUROPÄISCHE PATENTSCHRIFT

(45) Veröffentlichungstag der Patentschrift: **04.12.91**

(51) Int. Cl.[5]: **A61K 31/505**

(21) Anmeldenummer: **87104277.6**

(22) Anmeldetag: **24.03.87**

## (54) Cancerostatisches Mittel.

(30) Priorität: **04.04.86 DE 3611194**

(43) Veröffentlichungstag der Anmeldung:
**14.10.87 Patentblatt 87/42**

(45) Bekanntmachung des Hinweises auf die Patenterteilung:
**04.12.91 Patentblatt 91/49**

(84) Benannte Vertragsstaaten:
**AT BE CH DE ES FR GB IT LI NL SE**

(56) Entgegenhaltungen:

**LIEBIGS ANN. CHEM., Nr. 3, 1978, Seiten 398-404, Verlag Chemie GmbH, Weinheim, DE; H.-J. KABBE: "Heterocyclen aus Diaminen und Diacylverbindungen"**

**PROC. NATL. ACAD. SCI. USA, Band 49, 1963, Seiten 94-102, Washington, US, L.S. LERMAN: "The structure of the DNA-acridine complex"**

**DRUGS EXP. CLIN. RES., Band 14, Nr. 9, 1988, Seiten 571-574, Bioscience Ediprint Inc., CH; G. ATASSI et al.: "A new antitumour agent, batracylin, selected by a preclinical solid tumour model"**

(73) Patentinhaber: **BAYER AG**

**W-5090 Leverkusen 1 Bayerwerk(DE)**

(72) Erfinder: **Kabbe, Hans-Joachim, Dr.**
**Walter-Flex-Strasse 16**
**W-5090 Leverkusen 1(DE)**
Erfinder: **Bierling, Robert, Dr.**
**Merlinweg 18 b**
**W-5600 Wuppertal 1(DE)**
Erfinder: **Atassi, Ghanem, Prof.**
**Institut Jules Bordet Rue Héger-Bordet 1**
**B-1000 Brussel(BE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 240 829 B1

Rank Xerox (UK) Business Services

**Beschreibung**

Es ist bekannt, daß weitgehend flache, mehrcyclische Verbindungen antibakteriell oder cancerostatisch wirken können, weil sie häufig in der Lage sind, sich zwischen die Windungen der DNS-Helix einzulagern [L.S. Lerman, Proc. Nat. Acad. Sci. USA 49 94 (1963)]. Wir haben eine solche sogenannte intercalierende Wirkung auch für Verbindungen vermutet, die aus Diaminen und Anhydriden zugänglich sind [H.J. Kabbe, Liebigs Ann. Chem. 1978, 398]. Tatsächlich erwiesen sich jedoch diese Substanzen bei der Untersuchung in einer Reihe von Tumor-Testmodellen, z.B. Leukämie 1210, B 16-Melanom oder Lewis Lung Carcinom, als nahezu unwirksam.

Es wurde nun gefunden, daß eine der von uns früher synthetisierten Verbindungen, das 8-Amino-12-oxo-10,12-dihydroisoindolo[1,2-b]chinazolin (I) am Modell des Colon 38-Carcinoms an der Maus ausgezeichnete tumorhemmende Eigenschaften zeigt. Dieses Modell ist repräsentativ für die Wirkung gegen Colon- und Rektumkrebs des Menschen.

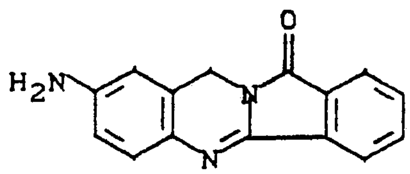

(I)

Dieses Ergebnis ist als außerordentlich überraschend zu bezeichnen, da bisher nur von sehr wenigen Verbindungen eine Wirkung gegen den Colon-Tumor bekannt geworden ist. (So wird bisher im wesentlichen nur das als Antimetabolit wirkende 5-Fluoruracil zur palliativen Behandlung dieser Tumore eingesetzt.)

Insbesondere zeigen die Verbindungen, die als intercalierend wirkend bekannt sind, in der Regel keine oder nur eine marginale Wirkung auf diesen speziellen Tumor. Die Auffindung der hohen Wirksamkeit gegen das Colon 38-Carcinom ist daher als ein wichtiger Fortschritt auf dem Krebsgebiet anzusehen. Besonders bemerkenswert ist die Tatsache, daß bereits eine zweimalige orale Applikation zur völligen Heilung führt.

Die Synthese der Wirksubstanz (I) ist in der genannten Arbeit (s.o.) angegeben. In den folgenden Beispielen werden die tumorhemmende Eigenschaft von (I) gegen Colon 38-Carcinom und das Fehlen der Colon 38-Wirkung mit Ellipticin als Vergleichssubstanz beschrieben.

Der Wirkstoff kann in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken des Wirkstoffes mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, physiologische Kochsalzlösung (1-prozentig), nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, kann der Wirkstoff außer mit den obengenannten Hilfsstoffen mit verschiedenen Geschmacksaufbesserern oder Farbstoffen versetzt werden.

In besonderen Fällen kann auch eine rektale Applikation vorteilhaft sein.

Für den Fall der parenteralen Anwendung können Lösungen des Wirkstoffes unter Verwendung geeigneter flüssiger Trägermaterialien eingesetzt werden.

Im allgemeinen hat es sich als vorteilhaft erwiesen, bei parenteraler Applikation Mengen von etwa 0,1 bis 10 g, vorzugsweise etwa 0,2 bis 5 g pro $m^2$ Körperoberfläche pro Tag zur Erzielung wirksamer Ergebnisse zu verabreichen, und bei oraler Applikation beträgt die Dosierung etwa 0,1 bis 20, vorzugsweise 0,2 bis 10 g pro $m^2$ Körperoberfläche pro Behandlungstag.

Die Behandlung kann entweder 1 bis 3 mal täglich oder in größeren Intervallen, z.B. einmal pro Woche, erfolgen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und dem individuellen Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungspielraum vorgesehen. Sinngemäß gelten hierbei auch die obigen Ausführungen.

Beispiel 1

Am Tage 0 wurden 70 mg von Colon-38-Tumorgewebe von C57BL6-Mäusen subcutan in das axillare Gebiet von B6C3F1-Mäusen implantiert (je 10 Tiere pro Dosis sowie 20 Tiere in der Kontrolle). An den Tagen 2 und 9 nach der Tumor-Implantation wurde die Verbindung (I) intraperitoneal appliziert (als 2 proz. Suspension in 1 proz. Kochsalzlösung unter Zusatz von ca. 1 % Tween 80). Am Tage 20 wurden die Tumorgewichte der behandelten und der unbehandelten Mäuse verglichen.

Folgende Resultate wurden erhalten:

| Dosis (mg/kg) | Durchschnittl. Tumorgewicht pro Maus nach 20 Tagen | |
|---|---|---|
| | in mg | in % zur Kontrolle |
| 200 (a) | 0 | 0 |
| 100 (b) | 0 | 0 |
| 50 (c) | 307 | 32 |
| Kontrolle | 936 | 100 |

Von den behandelten jeweils 10 Tieren konnten als völlig geheilt eingestuft werden: je 9 (a und b) bzw. 2 (c); substanzbedingte toxische Symptome traten in allen Behandlungsgruppen nicht auf.

Beispiel 2

200 mg der Wirksubstanz (I) wurden in 1 m1 1n-Salzsäure gelöst, die Lösung mit 9 ml sterilem Wasser verdünnt und anschließend intraperitoneal appliziert. Die Versuchsdurchführung erfolgte analog Beispiel 1.

| Dosis (mg/kg) | Durchschnittl. Tumorgewicht pro Maus nach 20 Tagen | |
|---|---|---|
| | in mg | in % zur Kontrolle |
| 100 | 0 | 0 |
| 50 | 252 | 28 |
| Kontrolle | 900 | 100 |

6 der 10 mit 100 mg/kg behandelten Tiere waren völlig geheilt. Substanzbedingte toxische Symptome traten in beiden Gruppen nicht auf.

Beispiel 3

Die Behandlung erfolgte oral anstatt intraperitoneal. Appliziert wurde die Substanz als Suspension an den Tagen 2 und 9, genau wie in Beispiel 1. Am Tage 20 wurden die Tumorgewichte bestimmt.

| Dosis (mg/kg) | Durchschnittl. Tumorgewicht pro Maus nach 20 Tagen | |
|---|---|---|
| | in mg | in % zur Kontrolle |
| 800 (a) | 0 | 0 |
| 600 (b) | 0 | 0 |
| 400 (c) | 2 | 0 |
| 200 (d) | 221 | 27 |
| Kontrolle | 798 | 100 |

Von den jeweils 10 behandelten Tieren wurden als völlig geheilt eingestuft: 9 (a), 8 (b), 5 (c) und 1 (d). Substanzbedingte toxische Symptome wurden nicht registriert.

Beispiel 4

B6C3F1-Mäusen wurde analog Beispiel 1 Colon 38-Tumorgewebe implantiert. Die Behandlung erfolgte mit 200 mg Ellipticin/kg [als 2 proz. Suspension in 1 proz. Kochsalzlösung unter Zusatz von ca. 1 % Tween 80] an den Tagen 2 und 9 (intraperitoneal), die Bewertung am Tage 20. Es wurden weder Heilungen noch eine Verminderung des Tumorgewichts im Vergleich zur Kontrolle beobachtet.

**Patentansprüche**

1. Cancerostatisches Mittel, gekennzeichnet durch einen Gehalt an 8-Amino-12-oxo-10,12-dihydroisoindolo[1,2-b]chinazolin der Formel (I)

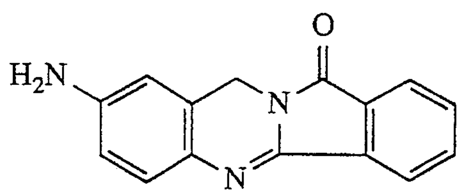

(I),

in inerten, nicht-toxischen, pharmazeutisch geeigneten Trägerstoffen.

2. Mittel zur Behandlung von Colon-und/oder Rektumkrebs des Menschen, gekennzeichnet durch einen Gehalt an 8-Amino-12-oxo-10,12-dihydroisoindolo[1,2-b]chinazolin in inerten, nicht-toxischen, pharmazeutisch geeigneten Trägerstoffen.

3. Mittel zur Behandlung des Colon-carcinoms, gekennzeichnet durch einen Gehalt an 8-Amino-12-oxo-10,12-dihydroisoindolo[1,2-b]chinazolin in inerten, nicht-toxischen, pharmazeutisch geeigneten Trägerstoffen.

4. Verfahren zur Herstellung eines cancerostatischen Mittels, dadurch gekennzeichnet, daß man 8-Amino-12-oxo-10,12-dihydroisoindolo[1,2-b]chinazolin mit inerten, nicht-toxischen, pharmazeutisch geeigneten Trägerstoffen vermischt.

5. Verwendung von 8-Amino-12-oxo-10,12-dihydroisoindolo[1,2-b]chinazolin der Formel (I)

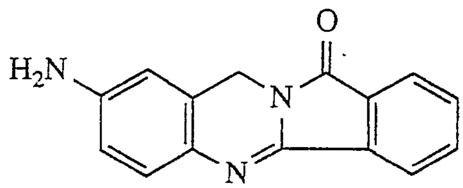

(I)

zur Herstellung von Arzneimitteln zur Bekämpfung von Colon-und/oder Rektumkrebs.

## Claims

1. Carcinostatic agent characterised by containing 8-amino-12-oxo-10,12-dihydroisoindolo[1,2-b]-quinazoline of the formula (I)

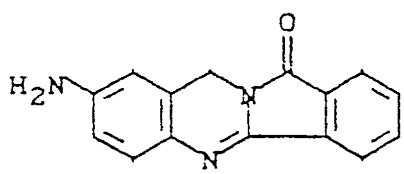

(I)

in inert, non-toxic, pharmaceutically suitable vehicles.

2. Agent for the treatment of cancer of the colon and/or rectum in humans, characterised by containing 8-amino-12-oxo-10,12-dihydroisoindolo[1,2-b]quinazoline in inert, non-toxic, pharmaceutically suitable vehicles.

3. Agent for the treatment of colon carcinoma, characterised by containing 8-amino-12-oxo-10,12-dihydroisoindolo[1,2-b]quinazolinein inert, non-toxic, pharmaceutically suitable vehicles.

4. Process for the preparation of a carcinostatic agent, characterised in that 8-amino-12-oxo-10,12-dihydroisoindolo[1,2-b]quinazoline is mixed with inert, nontoxic, pharmaceutically suitable vehicles.

5. Use of 8-amino-12-oxo-10,12-dihydroisoindolo-[1,2-b]quinazoline of the formula (I)

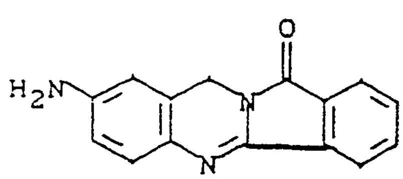

(I)

for the preparation of medicaments for combating cancer of the colon and/or rectum.

## Revendications

1. Agent cancérostatique **caractérisé par** une teneur en 8-amino-12-oxo-10,12-dihydroisoindolo[1,2-b]-quinazoline de formule (I)

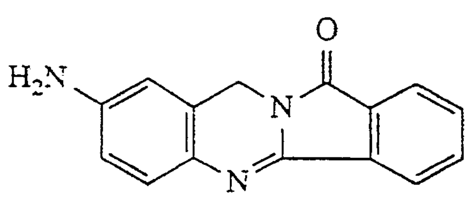

(I),

dans des substances de support inerte non toxiques pharmaceutiquement appropriées.

2. Agent pour le traitement du cancer du côlon et/ou du rectum chez l'être humain, **caractérisé par** une teneur en 8-amino-12-oxo-10,12-dihydroisoindolo[1,2-b]-quinazoline dans des substances de support inertes non toxiques pharmaceutiquement appropriées.

3. Agent pour le traitement du carcinome du côlon, **caractérisé par** une teneur en 8-amino-12-oxo-10,12-dihydroisoindolo[1,2-b]quinazoline dans des substances de support inertes non toxiques pharmaceutiquement appropriées.

4. Procédé pour la préparation d'un agent cancérostatique, **caractérisé en ce qu'**on mélange la 8-amino-12-oxo-10,12-dihydroisoindolo[1,2-b]quinazoline avec des substances de support inertes non toxiques pharmaceutiquement appropriées.

5. Utilisation de la 8-amino-12-oxo-10,12-dihydroisoindolo[1,2-b]quinazoline de formule (I)

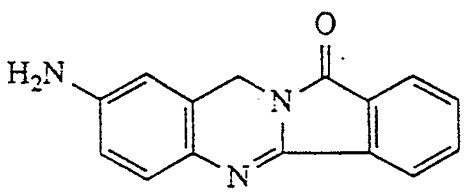

(I)

pour la préparation de médicaments destinés à lutter contre le cancer du côlon et/ou du rectum.